# EUROPEAN PATENT APPLICATION

(11) **EP 4 825 327 A1**
(43) Date of publication of application: **21.10.2026**
(21) Application number: 24828190.9
(22) Date of filing: 29.11.2024
(51) Int. Cl.: A61B 17/34

(54) **MEDICAL DEVICE FOR IMMOBILIZING A NEEDLE OR CATHETER**

(30) Priority: 13.12.2023 PT 2023119130
(71) Applicant: Bedev, 6200-284 Covilhã (PT)
(72) Inventor: DA FONSECA, Gonçalo Pereira, 6200-381 Covilhã (PT); GAMA, Maria Dolores Correia, 9125-014 Caniço (PT); LOPES, Mariana Santos Silva Daniel, 6200-087 Covilhã (PT)
(74) Representative: RCF - Protecting Innovation, S.A.
(86) International application number: PCT/PT2024/050044
(87) International publication number: WO 2025/127953

(57) **Abstract**

The present invention relates to a medical device (1) for immobilizing a needle or catheter during medical procedures, which comprises a body (2), a retaining cover (3) and a drive cover (4), wherein said body (2) is arranged with a plurality of gripping blades (6) and a rotating actuator (5), which is connected to said gripping blades (6). In turn, said retaining cover (3) is configured to axially retain the plurality of gripping blades (6) and the rotary actuator (5) inside the body (2). The drive cover (4) is arranged over the retaining cover (3) and connected to the rotary actuator (5) and is configured to, in use, provide alternative drive movements to the rotary actuator (5), which in turn alternatively provides an opening or closing of the gripping blades (6). The present invention also relates to a medical puncture kit and a paracentesis kit comprising the medical device (1).

## Description

### FIELD OF INVENTION

The present invention relates to a medical device (1) for immobilizing a needle or catheter, designed to assist medical professionals in procedures that require the precise and controlled application of these instruments, such as paracentesis and other similar procedures. The invention has application in the technical field of medical instruments, more specifically medical instruments for immobilizing needles or catheters.

### BACKGROUND OF THE INVENTION

In the medical field, there are several medical procedures that involve the application of needles or catheters to the patient for diagnostic, therapeutic or monitoring purposes. Some examples of these medical procedures include: diagnostic or therapeutic paracentesis, thoracentesis, image-guided biopsies, abscess drainage, central venous catheter insertion, cardiac catheterization, urinary catheter insertion, peripheral intravenous catheter insertion, epidural or intrathecal catheter insertion, hemodialysis catheter insertion and lumbar puncture. These are just a few examples of the many medical procedures that involve the application of needles or catheters to the patient.

Paracentesis is a medical procedure in which a needle or catheter is inserted into the abdominal region to drain fluid accumulated in the peritoneal cavity, the area between the abdominal walls and the internal organs. The main aim of paracentesis is to relieve symptoms such as abdominal distension, discomfort and difficulty breathing, which can occur due to the abnormal accumulation of fluid (ascites) in this region.

The paracentesis procedure is carried out by the doctor in a hospital or outpatient setting and follows certain steps that are generally similar in all hospitals or private clinics:
**1.** Preparing and positioning the patient: normally the patient is placed in a reclining, semi-sitting, sitting or fully lying position, with the abdominal region exposed.
**2.** Asepsis and local anesthesia: the needle or catheter insertion area is prepared and cleaned with antiseptic solutions and local anesthesia is administered.
**3.** Insertion of the needle or catheter: a needle and catheter are inserted through the abdominal wall into the peritoneal cavity; the needle is then removed and the catheter is left to guide the outflow of abdominal fluid.
**4.** Fluid drainage: as the catheter is advanced, the ascitic fluid begins to drain, and a bottle is attached at a lower height than the patient to drain the ascitic fluid, allowing the fluid to flow passively. The procedure is also often monitored using imaging techniques, such as ultrasound, to guide the insertion and monitor the amount of fluid drained.
5. Sample collection (Optional): During paracentesis, samples of ascitic fluid can be collected for laboratory analysis (if the procedure is of a diagnostic nature). These samples can help determine the underlying cause of the fluid accumulation.
6. Completion of the procedure: once the desired amount of fluid has been drained or the diagnostic or therapeutic purpose has been achieved, the needle or catheter is removed and a sterile dressing is applied to the site of the procedure.

In the paracentesis procedure described above, the needle or catheter is usually fixed by one of the following means:
- medical adhesive tape or gauze: the needle or catheter is fixed to the patient's skin using medical adhesive tape or gauze, which will help keep the instrument in place to minimize the risk of unwanted movements.
- manual fixation: in some cases, a member of the medical team holds the needle or catheter to provide the necessary stability during the procedure.
- medical sutures: help secure the needle or catheter in place.
- means of mechanical support: in some cases, special mechanical supports can be used to keep the needle or catheter stable during paracentesis, minimizing unwanted movements.

Properly securing the needle or catheter is an essential aspect of ensuring the accuracy and safety of the paracentesis procedure or any other similar medical procedure, with the main aim of minimizing the risks associated with inadvertent movements during medical procedures. Keeping the needle or catheter stable is one of the main concerns of the medical team carrying out these medical procedures.

The current means of securing needles or catheters have several drawbacks for both the patient and the healthcare professional. One of the main drawbacks is that they provide poor stability and fixation close to the patient's abdomen, which can lead to complications during medical procedures. Furthermore, for healthcare professionals, the current means of fixation do not allow the devices to be used during medical procedures or the needle or catheter to be inserted and removed easily without using non-invasive methods.

Document US5078688A refers to a paracentesis catheter system for removing fluids from a patient's abdominal cavity and for administering drugs into the abdominal cavity. As shown in figure 1 of document US5078688A, the system is divided into two main parts. The first part is understood to be the entire set of tubes and valves that allow the drained liquid (ascites) to be guided to the final collection container or for diagnosis. In turn, the second part is where the support device described will act, comprising all the equipment that will allow the fluid accumulated inside the body to be reached by means of a puncture. This puncture is carried out using a needle inserted into a rigid catheter. The tip of the needle is left uncoated to allow better penetration of the skin. When the needle is inserted into the cavity where the accumulated fluid is located, it is then removed and the aforementioned catheter is left in its place, serving as a guide for the fluid to the piping system. This type of system makes the procedure minimally invasive, using just one puncture as the only invasive means. However, a support system for the catheter is required, and currently a simple adhesive is used to fix it.

As a support system, a set of invasive skin systems are usually used in the majority of cases. Document US8454632B2 refers to one of these systems, describing an "anchor" that is inserted at the skin level through penetrations between adjacent skin layers, where the phalanges of the "anchor" interact with the most superficial layers of tissue and the central region resides in the region where the skin penetrations have occurred.

The state of the art describes different ways in which this anchoring can take place, such as in document US8425539B2, in which the type of anchoring found, as in the previous document, is carried out in a first phase, with a puncture allowing the device to penetrate the skin followed by the release of the mechanism. In this case, the anchoring is carried out using 4 arms which, as they penetrate the skin, extend and bend, creating a kind of staple anchoring in the outermost layers of the inner and outer skin or organ in question. Once fixed, the hollow stent in the center of the device allows the insertion of catheters or the passage of fluid accumulated in the region most adjacent to where the device was placed.

There are also robotic arm systems that are used in more advanced procedures, to position needles or catheters with precision. These systems provide a high degree of control, but are complex, expensive and are not widely adopted in all types of medical procedures, being used only in surgical settings.

Although there are various approaches and devices available for immobilizing needles or catheters in medical procedures, many of them have limitations in terms of precision, fit and support. Thus, there is a need for innovation in this area to improve the effectiveness and safety of medical procedures that involve the application of needles or catheters, and that are non-invasive.

### BRIEF DESCRIPTION OF THE FIGURES

The following is a detailed description of the invention with reference to the attached figures:
Fig. 1 illustrates the schematic exploded view of all the components of the medical device (1) for immobilizing a needle or catheter, according to the present invention, which comprises: the body (2), the retaining cover (3), the drive cover (4), the rotary actuator (5), the gripping blades (6) and the locking joint (7).
Fig. 2 is a schematic representation of the body (2) of the device, where (21) refers to the side wall, (22) corresponds to the open top, (23) is the bottom, (24) is the through opening, (25) refers to the cavity, (26) corresponds to the male-female fitting means with the (6) gripping blades and (27) refers to the male-female fitting means with the (3) retaining cover.
Fig. 3 is a schematic representation of the retaining cover (3) o, where (31) represents the through hole and (32) corresponds to the male-female fitting means with the body (2).
Fig. 4 is a schematic representation of the drive cover (4), where (41) corresponds to the through hole.
Fig. 5 shows the side view of the drive cover (4), where (42) corresponds to the male-female means of engagement with the rotary actuator (5).
Fig. 6 is the bottom view of the drive cover (4), i.e. its base, where (41) corresponds to the through hole and (42) corresponds to the male-female means of engagement with the rotary actuator (5).
Fig. 7 shows a schematic representation of the rotary actuator (5), where (51) represents the through hole and (52) corresponds to male-female plug-in means with the drive cover (4).
Fig. 8 shows the bottom view of the rotary actuator (5), i.e. its base, where (51) represents the through hole and (53) represents an eccentric pin-groove connection with the gripping blades (6).
Fig. 9 shows a schematic representation of the gripping blades (6), where (61) represents an eccentric pin-groove connection with the rotary actuator (5), and (62) refers to the male-female means of engagement with the body (2).
Fig. 10 is a schematic representation of the locking joint (7), where (71) stands for through hole.
Fig. 11 shows the sectional view of the medical device (1).

### SUMMARY OF THE INVENTION

The present invention relates to a medical device (1) for immobilizing a needle or catheter, comprising a body (2), a retaining cover (3) and a drive cover (4), in which
- the body (2) is delimited by a side wall (21), an open top (22) and a bottom (23) having a through opening (24), which together define a cavity (25) inside which a plurality of gripping blades (6) and a rotating actuator (5) are arranged, where said actuator (5) comprises a through hole (51) and is connected to the gripping blades (6),
- the retaining cover (3), comprising a through hole (31), is arranged on said open top (22) of the body (2) and is configured to axially retain the plurality of gripping blades (6) and the rotary actuator (5) inside the cavity (25) and
- the drive cover (4) has a through hole (41) and is arranged over the retaining cover (3) and connected to the rotary actuator (5),
wherein said drive cover (4) is configured to, in use, provide alternative drive movements to the rotary actuator (5), which in turn alternatively provides an opening or closing of the gripping blades (6).

In a preferred embodiment, said drive cover (4) is a rotary drive cover.

In another embodiment of the present invention, the medical device (1) also comprises a locking joint (7) between the rotary actuator (5) and the side wall (21) of the body (2).

In one aspect, at least 3 gripping blades (6) are arranged inside the cavity (25) of the body (2) of the device.

In another aspect, said drive cover (4) is connected to the rotary actuator (5) by connection means selected from the group consisting of threaded means, mechanical adhesive means, chemical adhesive means, magnetic attraction means and snap-on means.

In yet another aspect of the invention, the aforementioned drive cover (4) is connected to the rotary actuator (5) by male-female plug-in means, preferably of the pin-hole type.

In one respect, said rotary actuator (5) is connected to the gripping blades (6) via an eccentric pin-groove connection.

In another aspect of the invention, said retaining cover (3) is connected to the body (2) by connecting means selected from the group consisting of threaded means, mechanical adhesive means, chemical adhesive means, magnetic attraction means and snap-on means.

In yet another aspect, said retaining cover (3) is connected to the body (2) by means of male-female fittings, preferably of the pin-hole type.

The present invention also relates to a medical puncture kit comprising the medical device (1) of the invention, a needle and a catheter.

The present invention also relates to a paracentesis kit comprising the medical device (1) of the invention and a paracentesis needle.

### DETAILED DESCRIPTION OF THE INVENTION

The present invention relates to a medical device for immobilizing needles or catheters, designed to assist medical professionals in procedures that require the precise and controlled application of such instruments, such as paracentesis, thoracentesis and other similar medical procedures. The present invention also relates to a medical puncture kit and a paracentesis kit that comprise the medical device disclosed herein. The invention has application in the technical field of medical instruments, more specifically medical instruments for immobilizing needles or catheters.

In the context of the present invention, the term "gripping blades" refers to elements of small thickness in relation to their axial dimensions which are intended to act together, through a set of at least 3 blades, to hold or release a needle or catheter in operation, thus contributing to the desired effect of immobilizing them when the medical device is in use.

In turn, the term "rotary actuator" refers to an intermediate element for actuating these blades. This element is directly connected to the blades on one side, with the other side being connected to a medical device drive cover.

In addition, the term "actuation cap" refers to an element with which the user of the medical device interacts manually to actuate the aforementioned gripping blades.

The term "locking joint" refers to an element arranged in the cavity of the device body, between the rotary actuator and the side wall of the same body. In addition to suppressing the gap between the rotary actuator and the side wall of the same body, the locking joint prevents free movement of the rotary actuator and, consequently, of the gripping blades, helping to fix the gripping blades in the closed position around the needle or catheter, which can have different thicknesses or diameters.

The term "retaining cover" refers to an element intended to axially retain the gripping blade assembly, the locking joint and the rotary actuator inside the body of the device, the retaining cover being arranged on the open top of the body of the medical device.

In the context of this description, the term "comprising" should be understood as "including but not limited to". As such, it should not be interpreted as "consisting only of".

The device provides a stable and safe platform for the precise insertion and manipulation of needles or catheters, minimizing the risks associated with involuntary movements and improving the efficiency and precision of medical procedures.

This medical device can be applied to different areas of the body, such as the rib cage, lower back, abdomen, etc. and can be placed before or after the needle or catheter is inserted into the body.

The medical device of the present invention comprises several interconnected elements in which each plays a specific role to ensure the objectives of immobilization, stability and support of needles or catheters during a given medical procedure.

In one form of the present invention, the medical device of the present invention focuses on optimizing the paracentesis procedure, namely for better immobilization, support and stabilization of the catheter, which is left in the patient's abdomen for continuous extraction of the liquid (ascites) present in the abdomen. Thus, in the case of the medical procedure of paracentesis, the device of the present invention has the function of immobilizing the catheter after the needle has been inserted into the patient's abdomen. The device is placed under the surface of the skin, in the abdominal region of interest, and will allow the immobilization and stabilization of the catheter in the patient's abdomen after the entire procedure, which is left during the procedure to remove all the fluid accumulated in the abdomen. To date, this procedure has been carried out using gauze or adhesive to immobilize and stabilize the needle in the patient's abdomen, which have proven to be ineffective.

The medical device of the present invention is adaptable, since it is possible to apply it to medical procedures similar to paracentesis, namely thoracentesis which is based on a similar principle, the removal of pleural fluid.

The medical device of the present invention improves the paracentesis procedure and other similar medical procedures, as it contributes to a reduction in complications and greater ease of application for the healthcare professional. The medical device of the present invention makes the use of adhesive unnecessary, ensuring stability, immobilization and better fixation of the needle or catheter close to the abdomen or other region of the patient's body, reducing minor complications that can arise due to poor stability and immobilization of the needle or catheter, and allowing the healthcare professional to place and remove the needle or catheter with greater ease and safety.

Thus, the aim of the present invention is to improve medical procedures involving the application of needles or catheters in the patient's body, from a medical and technical point of view, contributing to a reduction in complications and greater ease of application by the health professional.

Contrary to the state of the art, the medical device of the present invention promotes the safety of medical procedures involving medical puncture, in a non-invasive way , also allowing easy replacement of each component of the device, without the need to replace the entire device, and allowing its reuse, requiring only sterilization and disinfection. Another advantage of the medical device of the present invention is that it can be adapted to different medical procedures in which there is a need to immobilize needles or catheters.

As mentioned above, the medical device of the present invention has several advantages over what currently exists:
- Reducing the rate of complications associated with infection by ensuring optimal aseptic conditions;
- It allows the equipment to be better adapted to each patient's individual biotype, particularly the characteristics of their abdominal wall;
- Acceleration of healing at the puncture site through a hypoallergenic adhesive that may contain an agent to aid healing;
- It facilitates the performance of paracentesis and similar medical procedures by healthcare professionals, contributing to a *user-friendly* application;
- Immobilization of the needle or catheter inserted into the abdomen, increasing the safety of the procedure itself;
- Allows the device to be adapted to different types of equipment;
- Produced in a material that allows sterilization by autoclaving and/or non-ionizing radiation (UV); and also
- Design that allows each element to be replaced individually in the event of damage or contact with bodily substances that make it impossible to reuse.

The medical device of the present invention will function during the period in which the needle or catheter is inserted into the abdominal region, or other region of the body where the medical procedure is being carried out, serving as a guide between the fluid accumulated in the abdomen, or other region of the body, and the drainage system.

The construction and assembly of the device itself makes it possible to replace any of its parts without having to dispose of the entire device. This makes it possible to replace specific parts in the event of damage and/or contact with body fluids, although it is possible to sterilize and disinfect the device.

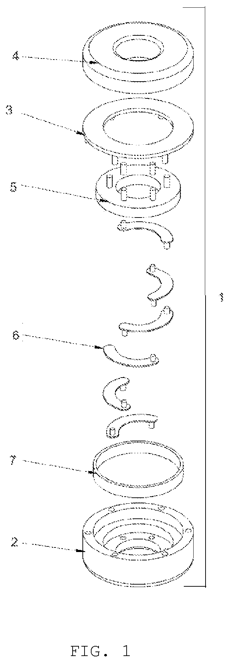

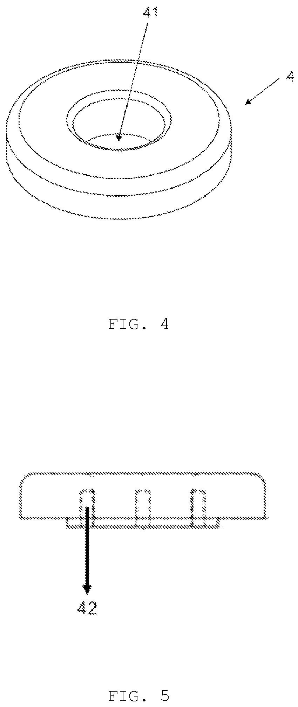

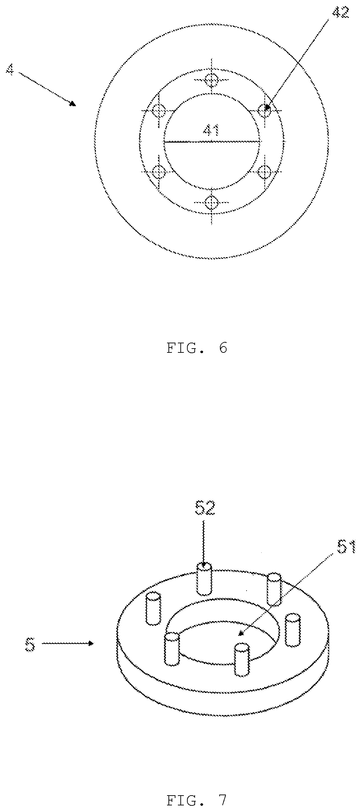

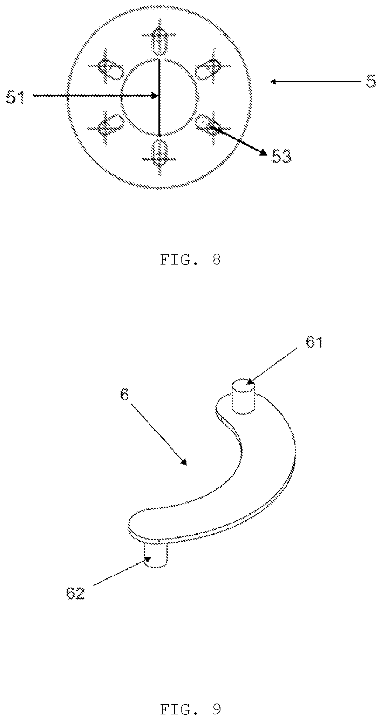

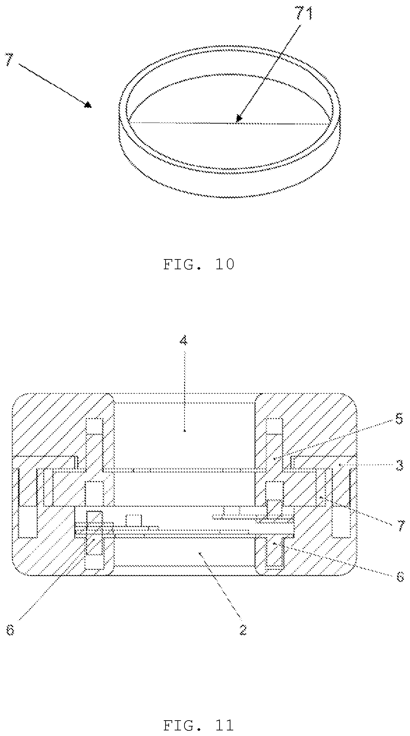

Thus, with reference to Figs. 1 to 11, which represent a preferred and merely illustrative embodiment of the invention, the medical device (1) comprises a body (2), a retaining cover (3) and a drive cover (4), wherein
- the body (2) is delimited by a side wall (21), an open top (22) and a bottom (23) having a through opening (24), which together define a cavity (25) within which a plurality of gripping blades (6) are arranged, a rotary actuator (5), in which said actuator (5) comprises a through hole (51) and is connected to the gripping blades (6), and also a locking joint (7), arranged between the rotary actuator (5) and the side wall (21) of the body (2),
- the retaining cover (3), comprising a through hole (31), is arranged on said open top (22) of the body (2) and is configured to axially retain the plurality of gripping blades (6), the locking joint (7) and the rotary actuator (5) inside the cavity (25) and
- the drive cover (4) has a through hole (41) and is arranged over the retaining cover (3) and connected to the rotary actuator (5), and
wherein said drive cover (4) is configured to, in use, provide alternative drive movements to the rotary actuator (5), which in turn alternatively provides an opening or closing of the gripping blades (6).

This locking joint (7) has the function of suppressing the gap between the rotary actuator (5) and the side wall (21) of the body (2), as well as sealing and creating friction between the rotary actuator (5) and the side wall (21) of the body (2), allowing the gripping blades (6) to be fixed through friction. Thus, the locking joint (7) fixes the rotary actuator (5) and consequently fixes the gripping blades (6), thus allowing the needle or catheter to be immobilized when the medical device is in use.

In one embodiment, the locking joint (7) is made of a rubber or silicone material or any other suitable material that creates friction between the rotary actuator (5) and the side wall (21) of the body (2), and a material that is sterilizable.

The aforementioned drive cover (4) is a rotary drive cover and is the part of the device (1) that the healthcare professional will operate to immobilize the needle or catheter during the medical procedure. The drive cover (4) corresponds to the upper part of the device (1), and is the part that the healthcare professional will turn and thus activate the gripping blades (6), in order to close or open them and, consequently, immobilize or not immobilize the needle or catheter, respectively.

Furthermore, said drive cover (4) is connected to the rotary actuator (5) by connection means selected from the group consisting of threaded means, mechanical adhesive means, chemical adhesive means, magnetic attraction means and snap-on means.

In one embodiment, the drive cover (4) is connected to the rotary actuator (5) by means of male-female fittings, preferably of the pin-hole type.

In another embodiment, the drive cover (4) is connected to the rotary actuator (5) via at least two pin-orifice assemblies.

In a preferred embodiment, the drive cover (4) is connected to the rotary actuator (5) by means of six pin-hole assemblies.

This rotary actuator (5) is connected to the gripping blades (6) via an eccentric pin-groove connection, allowing the gripping blades (6) to be moved, i.e. allowing the gripping blades (6) to be opened or closed to immobilize the needle or catheter and adapt the device to the size of the needle or catheter.

In a preferred design, the rotary actuator (5) is connected to the gripping blades (6) via an eccentric pin-groove connection. In this case, each blade has a pin that fits into a respective open slot in the rotary actuator (5). This means that, in the present preferred case of three blades, the rotary actuator comprises three slots.

In another form of realization, the medical device of the invention comprises six gripping blades (6).

Furthermore, said gripping blades (6) are connected to the device body (2) by means of male-female fitting means, preferably of the pin-hole type.

Since the rotary actuator (5) is connected to the gripping blades (6) via an eccentric pin-groove connection (61), it is possible to move the pins of each blade (6) after the rotary movement of the rotary actuator (5) (with the healthcare professional acting on the drive cover (4) with a rotary movement) to close and open the gripping blades (6).

Said retaining cover (3) is arranged on the open top (22) of the body (2) and is fixed to said body (2) by means of threaded means, mechanical adhesive means, chemical adhesive means, magnetic attraction means and snap-on means.

In a preferred embodiment, the retaining cover (3) is attached to the device body (2) by means of male-female fittings, preferably of the pin-hole type.

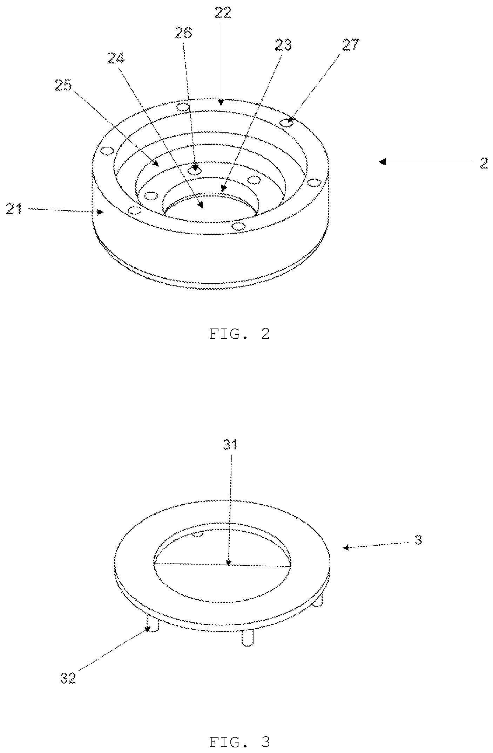

Figs. 2 to 10 show the aforementioned elements (2) to (7) of the medical device (1) of the present invention, which together form the medical device for immobilizing needles or catheters during the paracentesis procedure or similar procedures.

The body (2) consists of the base structure of the device (1) which is positioned over the patient's area of interest, providing a firm surface for immobilization. It is also adjustable and can be adapted to different sizes and anatomical positions, ensuring suitability for different patients and medical procedures.

In one aspect of the present invention, the construction and assembly of the device (1) makes it possible to replace the elements (2) to (7) individually, without the need to replace the entire device (1).

With reference to Figs. 1 to 11, in a preferred embodiment, the order of assembly of each of the aforementioned elements (2) to (7) of the medical device (1) is as described below:
- the body (2) serves as the base of the entire device (1) and is in direct contact with the skin;
- the gripping blades (6) are placed inside the cavity (25) of said body (2) by inserting the pin of the male-female inserting means (62) into the hole in the body (2);
- each gripping blade (6), when placed in each hole of the male-female means of engagement (26) by means of its male-female means of engagement pin (62), in sequential order, will be superimposed on the previously placed gripping blade (6), leaving the eccentric connection pin (61) of the pin-groove type of each blade (6) exposed for subsequent engagement with the rotary actuator (5);
- in turn, the rotary actuator (5) is connected to the gripping blades (6) by inserting the pin of the eccentric connection (61) of the pin-slot type of each blade (6) into the slots of the eccentric connection (53) of the pin-slot type of the rotary actuator (5);
- then the locking joint (7) is placed between the rotary actuator (5) and the side wall (21) of the body (2);
- the retaining cover (3) is arranged on the open top (22) of the body (2) and is fixed to said body (2) by means of a pin-hole type fitting, i.e. each pin of the male-female means of fitting (32) of the retaining cover (3) is placed in each hole of the male-female means of fitting (27) of the body (2);
- finally, the drive cover (4) is then arranged under the retaining cover (3) and, once the pins of the male-female means of engagement (52) of the rotary actuator (5) are exposed, the said pins of the male-female means of engagement (52) of the rotary actuator (5) are fitted into the holes of the male-female means of engagement (42) of the drive cover (4).

With the above assembly, the device (1) of the present invention is obtained, which is based on a rotating manual locking and opening system based on a rotating movement of the drive cover (4) in an anti-clockwise/clockwise direction in relation to the body (2) of the device. The rotating action of the drive cover (4) results in the joint movement of the rotary actuator (5) and the gripping blades (6), which in turn reduce the diameter of the through opening (24) and allow the needle or catheter to be fixed and immobilized.

Thus, to activate the rotary locking or opening system of the device (1), the user makes a counterclockwise or clockwise rotation of the drive cover (4), respectively. With this movement, the rotary actuator (5) moves together with the drive cover (4) and, consequently, the gripping blades (6) move from the open position to the closed position with the counterclockwise movement and from the closed position to the open position with the clockwise movement.

Fixing the medical device (1) to the patient's skin, specifically at the puncture site, is achieved through the use of an adhesive as a fixing agent, which is located at the base of the body (2) of the device (1). Furthermore, after the medical procedure has been completed, this adhesive can remain fixed to the patient's skin, in order to aid and promote better healing of the surgical insertion, reduce the rate of infection and reduce the material spent on post-operative treatment.

Since the device (1) of the present invention is for medical purposes, it is designed in a material whose composition allows sterilizations to be carried out without the device (1) losing its structural properties or becoming harmful to the user/patient. To this end, the device (1) is made from a material that is already used in hospitals, such as polylactic acid (PLA) or polyethylene terephthalate (PET). These materials are already used in scalpel handles and plastic packaging respectively, which makes the manufacturing process easier as they are already used in various industries, including the production of various medical devices.

The industrial production of the device (1) of the present invention with the type of materials mentioned above is based on the technology of injecting molten plastic into a mold with the shape of the desired part. This process allows the production of thousands of units per day. As it is a medical device, post-production care, such as sterilization and vacuum packaging, is taken into account and the regulatory standards of each country are followed.

Thus, in view of the above, the device (1) of the present invention consists of an innovative solution that has several significant advantages compared to conventional techniques for fixing needles or catheters, namely greater precision, reduced risk of complications arising from involuntary movements and provides a controlled environment for medical procedures. In addition, the device's adaptability to different patients and medical procedures makes it a versatile and valuable tool for medical professionals in various specialties.

The device (1) is particularly useful in medical procedures such as paracentesis, image-guided biopsies, insertion of catheters for drainage and infusion, among others. Its applicability covers a variety of medical fields where precision in the application of needles or catheters is crucial.

In conclusion, the invention presents a remarkable advance in the field of medical devices, providing an effective and efficient solution for immobilizing needles or catheters during medical procedures and its ability to improve the accuracy and safety of procedures has the potential to benefit both medical professionals and patients.

It should be noted that although the present invention has been described with reference to its preferred forms of realization, many modifications and alternatives can be made by a person skilled in the art without leaving the scope of the invention, which is defined by the claims.

## Claims

1. A medical device (1) for immobilizing a needle or catheter, the device (1) **characterized by** comprising a body (2), a retaining cover (3) and a drive cover (4), wherein
- the body (2) is delimited by a side wall (21), an open top (22) and a bottom (23) having a through opening (24), which together define a cavity (25) inside which a plurality of gripping blades (6) are arranged, and a rotating actuator (5), where said actuator (5) comprises a through hole (51) and is connected to the gripping blades (6),
- the retaining cover (3), comprising a through hole (31), is arranged on said open top (22) of the body (2) and is configured to axially retain the plurality of gripping blades (6) and the rotary actuator (5) inside the cavity (25) and
- the drive cover (4) has a through hole (41) and is arranged over the retaining cover (3) and connected to the rotary actuator (5),
wherein said drive cover (4) is configured to, in use, provide alternative drive movements to the rotary actuator (5), which in turn alternatively provides an opening or closing of the gripping blades (6).

2. The medical device (1) according to claim **1, characterized in that** said drive cover (4) is a rotary drive cover.

3. The medical device (1) according to claim 1 or **2, characterized in that** it further comprises a locking joint (7) arranged between the rotary actuator (5) and the side wall (21) of the body (2).

4. The medical device (1) according to any of claims 1 to **3, characterized in that** at least 3 gripping blades (6) are arranged inside the cavity (25) of the body (2) of the device.

5. The medical device (1) according to any one of claims 1 **to 4, characterized in that** said drive cover (4) is connected to the rotary actuator (5) by connection means selected from the group consisting of threaded means, mechanical adhesive means, chemical adhesive means, magnetic attraction means and snap-on means.

6. The medical device (1) according to claim **5, characterized in that** said drive cover (4) is connected to the rotary actuator (5) by means of male-female fitting means, preferably of the pin-hole type.

7. The medical device (1) according to any of the preceding claims 1 to **6, characterized in that** the rotary actuator (5) is connected to the gripping blades (6) by means of an eccentric pin-groove connection.

8. The medical device (1) according to any one of claims 1 to 7, **characterized in that** said retaining cover (3) is connected to the body (2) by connecting means selected from the group consisting of threaded means, mechanical adhesive means, chemical adhesive means, magnetic attraction means

9. The medical device (1) according to claim 8, **characterized in that** said retaining cover (3) is connected to the body (2) by means of a male-female fitting, preferably of the pin-hole type.

10. A medical puncture kit, **characterized in that** it comprises the medical device (1) claimed in any of the preceding claims 1 to 9, a needle and a catheter.

11. A paracentesis kit **characterized in that** it comprises the medical device (1) claimed in any one of claims 1 to 9 and a paracentesis needle.
